(19) Europäisches Patentamt
European Patent Office
Office européen des brevets

(11) EP 0 887 332 B1

(12) **EUROPÄISCHE PATENTSCHRIFT**

(45) Veröffentlichungstag und Bekanntmachung des Hinweises auf die Patenterteilung:
**27.12.2000 Patentblatt 2000/52**

(51) Int Cl.[7]: **C07C 41/08**, B01J 23/06, C07C 41/54, C07C 43/16, C07C 43/303

(21) Anmeldenummer: **98110452.4**

(22) Anmeldetag: **08.06.1998**

(54) **Verfahren zur Addition von Hydroxylgruppen enthaltenden Verbindungen an Alkine oder Allene**

Process for the addition of hydroxyl group-containing compounds to alkynes or allenes

Procédé pour l'addition de composés contenant un groupe hydroxyle sur les alcynes ou les allènes

(84) Benannte Vertragsstaaten:
**CH DE FR GB IT LI**

(30) Priorität: **23.06.1997 DE 19726668**

(43) Veröffentlichungstag der Anmeldung:
**30.12.1998 Patentblatt 1998/53**

(73) Patentinhaber: **BASF AKTIENGESELLSCHAFT**
**67056 Ludwigshafen (DE)**

(72) Erfinder:
- **Teles, Joaquim Henrique, Dr.**
  **67122 Altrip (DE)**
- **Rieber, Norbert, Dr.**
  **68259 Mannheim (DE)**
- **Breuer, Klaus, Dr.**
  **67122 Altrip (DE)**
- **Demuth, Dirk, Dr.**
  **68161 Mannheim (DE)**
- **Hibst, Hartmut, Prof.Dr.**
  **69198 Schriesheim (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:
- **CHEMICAL ABSTRACTS, vol. 112, no. 7, 12. Februar 1990 Columbus, Ohio, US; abstract no. 54986a, O. N. TEMKIN: "A catalyst for the manufacture of methyl and ethyl isopropenyl ethers" Seite 687; Spalte 1; XP002080414 & DD 267 629 A**
- **CHEMICAL ABSTRACTS, vol. 112, no. 7, 12. Februar 1990 Columbus, Ohio, US; abstract no. 54987b, O. N. TEMKIN: "A catalyst for the manufacture of methyl and ethyl isopropenyl ether" Seite 687; Spalte 1; XP002080415 & DD 265 289 A**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 887 332 B1

Printed by Jouve, 75001 PARIS (FR)

## Beschreibung

Die Erfindung betrifft ein Verfahren zur Addition von Hydroxylgruppen enthaltenden Verbindungen an Alkine oder Allene unter Bildung von Aldehyden und Ketonen oder deren Derivaten in Form von Enolethern oder Acetalen bzw. Ketalen in Gegenwart eines amorphen Zink- oder Cadmiumsilikat-Katalysators. Ferner betrifft die Erfindung ein neues Verfahren zur Herstellung eines Zink- oder Cadmiumsilikats und den so erhaltenen Katalysator.

Die Addition von Hydroxylgruppen enthaltenden Verbindungen an Alkine bzw. Allene wird fast ausnahmsweise mit homogen gelösten Katalysatoren durchgeführt, z. B. mit Säuren, Basen und Übergangsmetallkomplexen (vgl. Houben-Weyl, Methoden der organischen Chemie, Bd 6/3, S. 233, S. 90, Bd. 5/2a, S. 738, Bd. 6/ld, S. 136 und Bd. 7/a, S. 816).

Die Säurekatalyse ist meistens auf die Addition an aktivierte, elektronenreiche Alkine (wie Acetylenether, R-C≡C-OR', Acetylenthioether, R-C≡C-SR' und Acetylenamine, $R\text{-}C{\equiv}C\text{-}NR'_2$) beschränkt.

Basenkatalysiert (in Gegenwart von KOH oder Alkoholat) können Alkohole in der Flüssigphase auch an nicht aktivierte Alkine addiert werden. Dies ist die gebräuchlichste Methode; allerdings bedarf die Reaktion hoher Temperaturen und Drücke und die Raumzeitausbeute ist relativ gering. Typischerweise benötigt man für die basenkatalysierte Vinylierung eines Alkoholes zwischen 6 und 10 h Verweilzeit bei ca. 160°C und 18 bis 20 bar Druck.

Die Addition kann auch durch Übergangsmetallkomplexe in der Flüssigphase katalysiert werden. Für die Addition von Alkoholen eignen sich insbesondere Quecksilber(II)- oder Gold(I)-Salze, während für die Addition von Carbonsäuren und Phenolen Zink- und Cadmiumsalze bevorzugt werden.

Die Addition von Carbonsäuren (insbesondere Essigsäure und Propionsäure) an Acetylen kann auch in der Gasphase in Gegenwart entsprechender Zinkcarboxylate (auch basischer Zinkcarboxylate gemäß CH 239 752) auf oberflächenreichen Trägern als Katalysatoren durchgeführt werden.

Schließlich ist auch bereits die Addition von Methanol an Propin bzw. Propadien in der Gasphase in Gegenwart von Zinkoxid auf Aktivkohle oder Silikagel in DD 265 289 bzw. von Zinknitrat auf Aktivkohle oder Silikagel in DD 267 629 bei Temperaturen oberhalb von 200°C beschrieben.

Alle diese Verfahren des Standes der Technik haben Nachteile. Sie haben entweder nur ein beschränktes Anwendungsgebiet oder benötigen, wie die basenkatalysierte Addition, hohe Drücke und Temperaturen, was zu Sicherheitsproblemen führen kann, oder sie haben nur eine geringe Raumzeitausbeute. Homogen gelöste Übergangsmetallkatalysatoren sind oft nach einer geringen Anzahl von Zyklen desaktiviert und sind außerdem schwierig zurückzuführen. Heterogene Katalysatoren für die Addition an Alkine bzw. Allene sind bisher nur selten beschrieben worden. Zink- bzw. Cadmiumcarboxylate auf Aktivkohle katalysieren lediglich die Addition von Carbonsäuren (z. B. Essigsäure oder Propionsäure) an Acetylen. Der o. g. Katalysator auf der Basis von Zinkoxid auf Aktivkohle oder Silicagel (DD 265 289) ist zwar in der Lage die Addition von Alkoholen (Methanol oder Ethanol) an Propin bzw. Propadien mit guter Selektivität (90 bis 96 %) zu katalysieren, die katalytische Aktivität ist aber relativ gering und die benötigten Reaktionstemperaturen und Kontaktzeiten sind hoch, was zu einer raschen Desaktivierung des Katalysators führt. Zinknitrat auf Aktivkohle oder Silicagel ist auch bei der in DD 267 629 angewandten Temperatur von über 200°C ungefähr eine Größenordnung weniger aktiv als das Zinkoxid und die Selektivität ist viel geringer (max. 70 %).

Es bestand daher die Aufgabe, einen möglichst bei niedrigen Temperaturen, d.h. Temperaturen unter 200°C, aktiven und selektiven heterogenen Katalysator für die Addition von Hydroxylgruppen enthaltende Verbindungen an Alkine, Allene oder Gemische davon vorzuschlagen.

Diese Aufgabe wurde erfindungsgemäß gelöst mit einem Verfahren zur Herstellung von Verbindungen der Formeln I bzw. II

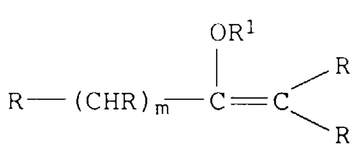
I

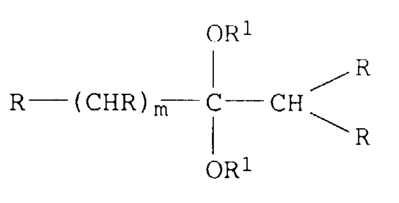
II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können und die Reste R unabhängig voneinander für Wasserstoff, oder aliphatische, cycloaliphatische, araliphatische, aromatische oder heterocyclische Reste stehen, die unter Bildung eines Ringes miteinander verbunden sein können und m für 0 oder 1 steht, durch Addition von Verbindungen der Formel III

$$R^1OH \qquad III$$

an Acetylene oder Allene der Formeln IV bzw. V

$$R-C\equiv C-R \qquad IV$$

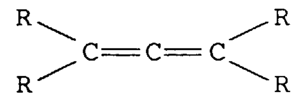 V

wobei $R^1$ und R die oben angegebenen Bedeutungen haben, in der Gasphase bei erhöhter Temperatur in Gegenwart eines heterogenen, durch Imprägnieren von Kieselsäure mit einem anorganischen Salz eines Elements der Gruppe 12 des Periodensystems der Elemente erhaltenen Katalysators, dadurch gekennzeichnet, daß man die Umsetzung bei einer Temperatur unterhalb von 200°C durchführt und einen Katalysator verwendet, der als aktiven Bestandteil ein röntgenamorphes Zinksilikat oder Cadmiumsilikat mit einem Gehalt an Zink bzw. Cadmium, berechnet als Oxid, von 1 bis 40 Gew.-% und z.B. eine BET-Oberfläche von 10 bis 1000 $m^2/g$ aufweist, erhältlich durch Aufbringen eines Salzes von Zink oder Cadmium und einer anorganischen Oxosäure, insbesondere Salpetersäure, das bei Temperaturen unterhalb von 400°C zersetzbar ist, auf amorphe Kieselsäure und Formierung des Katalysators vor der Umsetzung bei Temperaturen von 50 bis 500°C oder während der Umsetzung in situ bei Temperaturen von 50 bis 200°C in einer Gas/Festkörperreaktion, in Gegenwart einer Hydroxylgruppen enthaltenden Verbindung, ausgewählt aus der Gruppe bestehend aus Wasser, Alkanolen mit 1 bis 6 C-Atomen, Diolen oder Polyolen mit 2 bis 6 C-Atomen und 2 bis 3 OH-Gruppen, oder Carbonsäuren mit 1 bis 6 C-Atomen.

Die erfindungsgemäß zu verwendenden Katalysatoren können mit bis zu 80, vorzugsweise bis zu 20 Molprozent noch mit weiteren Metallen dotiert sein, ausgewählt aus der Gruppe (A) bestehend aus Natrium, Kalium, Lithium, Cäsium, Beryllium, Magnesium, Calcium, Strontium, Barium, Mangan, Eisen, Kobalt, Nickel und Kupfer, und der Gruppe (B), bestehend aus Titan, Zirkon, Hafnium, Germanium, Zinn und Blei.

Nach einer bevorzugten Ausführungsform des Verfahrens verwendet man amorphes Zinksilikat und stellt 2-Methoxypropen durch Addition von Methanol an Propin und/oder Allen her.

Als Edukte für die erfindungsgemäße Umsetzung kommen beliebige Alkine oder Allene oder Gemische davon in Betracht. In der Regel wird man jedoch technisch leicht zugängliche Acetylene und Allene mit 2 bis 8 C-Atomen, bzw. 3 bis 8 C-Atomen verwenden.

Die Hydroxylgruppen enthaltende Verbindung $R^1OH$ kann Wasser, ein beliebiger Alkohol, ein Phenol oder eine Carbonsäure sein. Im allgemeinen kommen vor allem Alkohole, besonders Alkanole mit 1 bis 16 C-Atomen, einkernige Phenole und niedermolekulare Carbonsäuren, z. B. mit 1 bis 16 C-Atomen, in Betracht.

Für die Formierung des Katalysators verwendet man zweckmäßig das gleiche Alkanol $R^1OH$, das man an das Acetylen bzw. Allen addiert.

Die Addition der Hydroxylgruppen enthaltenden Verbindungen erfolgt in Gegenwart des heterogen vorliegenden Katalysators in der Gasphase entweder über einem Festbett oder in einem Wirbelbett bei Temperaturen von 50 bis 200°C, vorzugsweise 100 bis 200°C und besonders bevorzugt 120 bis 200°C und Drücken von 0,1 bis 100 bar, insbesondere 0,8 bis 20 bar (alle Drücke bezogen auf Summe der Partialdrücke der Edukte).

Gegebenenfalls kann das Reaktionsgemisch aus Gründen der Betriebssicherheit und der besseren Wärmeabfuhr mit Inertgasen wie Stickstoff, Argon, niedermolekularen Alkanen oder Olefinen verdünnt werden.

Das molare Verhältnis zwischen der Hydroxylgruppen enthaltenden Komponente und Alkin bzw. Allen kann zwischen 0,01 und 100 liegen. Bevorzugt wird der Bereich zwischen 0,1 und 5, und besonders bevorzugt wird der Bereich zwischen 0,7 und 1,3 gewählt.

Durch die Reaktionsbedingungen kann die Selektivität der Reaktion bezüglich der Mono- bzw. Diadditionsprodukte gesteuert werden. Niedrige Verhältnisse zwischen der Hydroxylgruppen enthaltenden Komponente und Alkin bzw. Allen sowie hohe Temperaturen und niedrige Partialdrücke der Reaktanden führen zur bevorzugten Bildung der Monoadditionsprodukte während hohe Verhältnisse zwischen der Hydroxylgruppen enthaltenden Komponente und Alkin bzw. Allen sowie niedrige Temperaturen und hohe Partialdrücke der Reaktanden die Bildung der Bisadditionsprodukte begünstigen. Beispielsweise kann aus Propin oder Allen mit Methanol je nach Reaktionsbedingungen selektiv 2-Methoxypropen oder 2,2-Dimethoxypropan gebildet werden

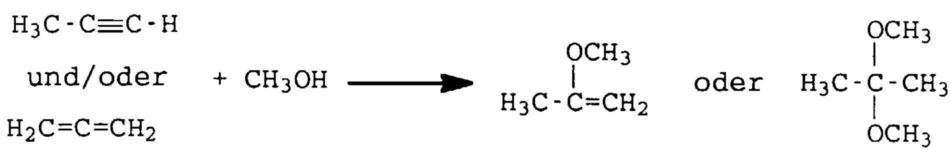

und/oder

oder

Zur Herstellung des Katalysators wird der amorphe $SiO_2$-Träger mit einer Lösung, zweckmäßig einer wäßrigen Lösung, der Zink- oder Cadmiumnitrate imprägniert. Eine Vermischung des trockenen oder in den Alkoholen $R^1OH$ suspendierten Nitrate mit dem Träger ist zwar möglich aber nicht vorteilhaft.

Der $SiO_2$-Träger ist zumindest weitgehend amorph, hat eine BET-Oberfläche zwischen 10 und 1500 $m^2/g$, bevorzugt 100 bis 500 $m^2/g$, eine Wasseraufnahmekapazität von 0,1 bis 2 ml/g, bevorzugt von 0,7 bis 1,3 ml/g und kann als Pulver oder als fertiger Formkörper eingesetzt werden. Der Träger kann auch vor der Imprägnierung kalziniert werden. Bevorzugt wird der Träger aber nicht kalziniert.

Von den aufzubringenden anorganischen Salzen sind die Nitrate und insbesondere das Zinknitrat vor dem Cadmiumnitrat bevorzugt.

Die Tränkung erfolgt nach an sich bekannten Methoden der Katalysatorherstellung. Wenn aus Löslichkeitsgründen erforderlich, kann die Beladung mit dem Aktivmetall auch in mehreren aufeinander folgenden Tränkschritten erfolgen.

Wenn der Träger als Pulver eingesetzt wird, kann er vor der Formierung durch Formgebung (z.B. durch Mischen, Kneten und Verstrangen oder Tablettieren) in die gewünschte Form gebracht werden.

Zur Erhöhung des Porenvolumens können bei der Formgebung auch Porenbildner zugesetzt werden (z.B. Superabsorber wie Lutexal P® (Firma BASF AG) oder Walocel® (Methylcellulose/Kunstharz-Kombination, Firma Wolff, Walsrode AG)).

Alternativ kann auch ein anderer Träger, z.B. $Al_2O_3$ zusammen mit einer Siliziumoxid-Vorläuferverbindung (z.B. $Si(OR)_4$) und mit einem Zinksalz imprägniert werden.

Die Zink- bzw. Cadmiumbeladung berechnet als Oxide kann in weiten Grenzen z.B. zwischen 1 und 40 Gew.-% variieren. Bevorzugt werden Zink- bzw. Cadmiumgehalte zwischen 7 und 30 Gew.-% und besonders bevorzugt zwischen 10 und 25 Gew.-%. Der so erhaltene, noch nicht aktive Präkatalysator kann dann bei einer Temperatur von maximal 600°C an Luft oder unter Inertgas kalziniert werden. Bevorzugt werden Kalziniertemperaturen zwischen 80 und 300°C. Besonders bevorzugt ist die Kalzinierung zwischen 120 und 250°C an Luft. Dabei wird zweckmäßig bei der Kalzinierung darauf geachtet, daß man Temperatur und Verweilzeit so wählt, daß das Molverhältnis des noch vorhandenen Anions zu Zink bzw. Cadmium 0,1 nicht unterschreitet.

Nach der Herstellung des Präkatalysators wird vor der Einbringung in den Reaktor oder in situ im Reaktor eine Formierung durchgeführt, bei der sich bevorzugt auf der Oberfläche des Katalysators die eigentliche Aktivphase ausbildet. Diese Gas/Festkörperreaktion wird bei einer Temperatur zwischen 80 und 500°C durch die Anwesenheit von Wasser, Alkoholen, bevorzugt niederen Alkoholen oder Carbonsäuren, bevorzugt niederen Carbonsäuren gefördert. Bevorzugt ist die Formierung des Katalysators zwischen 100 und 250°C in einem wasser- oder methanolhaltigen Gasgemisch und besonders bevorzugt zwischen 130 und 200°C mit einem methanolhaltigen Gasgemisch in situ im Reaktor, in dem die Umsetzung mit dem Alkin oder Allen stattfindet. Zweckmäßigerweise wird der Präkatalysator zur Ausbildung der Aktivphase unter Reaktionsbedingungen mit einem Gemisch aus Methanol und Propin und Allen und gegebenenfalls auch noch anderen inerten Komponenten, wie z.B. Propen oder Propan umgesetzt. Die Bildung der Aktivschicht wird durch das Ansteigen des Propin- und Allen-Umsatzes (nach ca. 5 bis 30 min, je nach Temperatur) und durch das Abklingen der Konzentration von Methylacetat im Abgas angezeigt. Ein stationären Zustand und eine hohe Selektivität wird je nach Temperatur nach ca. 2 bis 20 Stunden erreicht.

Für die Charakterisierung der Katalysatorproben, (frische, als auch Ausbauproben) wurden Standardmethoden verwendet. Die gemessene BET-Oberfläche (in der Regel zwischen 10 und 800 $m^2/g$) sowie die Härte sind bei dem jeweiligen Beispiel angegeben. Bevorzugt werden Katalysatoren mit BET-Oberflächen zwischen 100 und 400 $m^2/g$ verwendet. Weiterhin wurden die Proben mittels Pulverröntgendiffraktometrie (XRD) und Transmissionselektronenmikroskopie (TEM) eingehend untersucht. Mit beiden strukturaufklärenden Methoden ist keine Fernordnung im Sinne einer kristallinen Struktur festzustellen, sämtliche Proben waren amorph. Die Verteilung des Zinks über dem Träger wurde an Schnitten im Elektronenmikroskop sowie in der Mikrosonde untersucht. Sämtliche Proben zeigten, auch nach Ausbau, daß der Katalysator eine weitgehend homogene Elementverteilung besitzt und kein oder nur wenig kristallines ZnO enthält. Im $^{29}Si$-CP-MAS-NMR zeigte der Katalysator nur die breite Bande bei -109 ppm typisch für das amorphe $SiO_2$ und eine Schulter bei -99 ppm (ca. 15 % der Intensität des Hauptpeaks). Die Elementaranalyse eines Zn-Nitrat/$SiO_2$- Präkatalysators zeigte, daß das molare Verhältnis Nitrat/Zn von der Kalzinierungstemperatur abhängt. Bei Raumtemperatur getrocknete Katalysatoren haben eine Nitrat/Zn-Verhältnis von 1,6 - 1,9. Nach Calcinierung bei 120°C liegt das Nitrat/Zn-Verhältnis zwischen 1,0 und 1,5. Nach der Kalzinierung im bevorzugten Temperaturbereich von 200 bis

250°C liegt das Nitrat/Zn-Verhältnis zwischen 0,5 und 1. Bei höheren Temperaturen sinkt das Nitrat/Zn-Verhältnis noch weiter und ebenso die katalytische Aktivität der daraus gebildeten Katalysatoren.

Der Katalysator kann für die Umsetzung fest angeordnet sein oder z.B. auch in einem Wirbelbett verwendet werden und dafür eine entsprechende Gestalt haben, wie Splitt, Tabletten, Monolithe, Kugeln oder Extrudate (Stränge mit Querschnitten wie Vollstrang, Wagenrad, Stern oder Ring).

a) Allgemeine Umsetzungsbedingungen

Die katalytischen Umsetzungen gemäß Fig 1. wurden in einem gradientenfreien CSTR (Continuously Stirred Tank Reactor) (A) mit einem Volumen von 1740 ml und einem Katalysatorvolumen von ca. 90 ml, modifiziert für heterogene Gasphasenreaktionen durchgeführt. Der Reaktor hatte einen Innendurchmesser von ca. 108 mm und eine Höhe von ca. 200 mm und wurde mittels einer an der Innenwand angebrachten elektrischen Heizspirale beheizt. In der Mitte des Reaktors war ein kleiner Zylinder aus Metall angebracht (∅ ca. 64 mm, Höhe ca 150 mm), der auf halbe Höhe (ca. 85 mm unterhalb der Oberkante) mit einem Drahtgitter versehen war. Auf dieses Drahtgitter wurde der Katalysator locker aufgeschüttet. Auf dem Deckel des Reaktors war eine flache (∅ ca. 100 mm, Höhe ca. 20 mm) Turbine angebracht, die mit 1500-2000 Upm angetrieben wurde. An der Reaktorachse waren auf verschiedenen Höhen insgesamt 6 Thermoelemente zur Temperaturkontrolle angebracht. Die Edukte wurden unter Druck mittels HPLC-Pumpen dosiert, kurz vor dem Reaktor gemischt und in den Reaktorraum entspannt. Das Alkin bzw. Allen (1 in Fig 1) wurden entweder in reiner Form zudosiert oder als Gemisch mit anderen inerten Komponenten verdünnt. In dem Fall von Propin und Allen wurde ein Gemisch mit anderen Kohlenwasserstoffen eingesetzt (Zusammensetzung: 30-43 Vol% Propin, 16-20 Vol% Allen, 20-45 Vol% Propen, 5-10 Vol% Isobutan und 2-6 Vol% Propan als Hauptkomponenten; alle anderen Komponenten unter 1 %. Dieses Gemisch wurde durch Destillation aus einem Seitenstrom eines Steamcrackers gewonnen). Der Alkoholkomponente (2 in Fig 1) wurden ca. 10 Gew.-% Cyclohexan als interner Standard für die GC-Analytik zudosiert.

Die Reaktion wurde isotherm bei Temperaturen von 120 bis 200°C und einer Zulaufrate von 0,5 bis 10 mmol/min Propin und/oder Allen und 0,5 bis 20 mmol/min MeOH durchgeführt. Der Reaktionsdruck betrug 1,1 bis 3,5 bar (absolut).

Die Gesamtgasmenge, bestehend aus Edukten, Inertgas und internem Standard, betrug in der Regel zwischen 4 und 60 N1/h. Die GHSV (gas hourly space velocity), die definiert ist als

$$\text{GHSV} = \text{Gasvolumen [Nl/h]/Katalysatorvolumen} \qquad [1]$$

betrug zwischen 80 und 1200 $h^{-1}$. Die LHSV (liquid hourly space velocity), die definiert ist als

$$\text{LHSV} = \text{Flüssigkeitsvolumen [Nl/h]/Katalysatorvolumen} \qquad [1]$$

(hier gefördertes Volumen an Propin und MeOH Volumen) betrug zwischen 0,2 und 3 $h^{-1}$. Die Verweilzeit, definiert als Quotient aus dem Katalysatorvolumen [1] und der Gasmenge [Nl/s] betrug zwischen 3 und 40 s.

Die Reaktionsgase wurden nach Verlassen des Reaktors über eine beheizte Transferleitung (3) zu einem Online Gaschromatographen (B) geführt und dort alle 2 h analysiert. Danach wurde der Gasstrom einer partiellen Kondensation (C) unterworfen und der bei Raumtemperatur nicht kondensierbare Anteil (6) wurde in regelmäßigen Abständen (ca. 12 h) mittels Off-line GC analysiert. Das Kondensat (5) wurde ebenfalls gesammelt und mittels Off-line GC analysiert.

Wenn nichts anderes vermerkt, wurden die Umsätze und Selektivitäten auf die Summe von Propin und Allen bezogen.

b) Methoden zur Charakterisierung der Katalysatoren

Für die Charakterisierung der Katalysatorproben (frische als auch Ausbauproben) wurden Standardmethoden verwendet. Die gemessene BET-Oberfläche sowie die Härte sind bei dem jeweiligen Beispiel angegeben. Weiterhin wurden die Proben mittels Pulverröntgendiffraktometrie (XRD) und Transmissionselektronenmikroskopie (TEM) eingehend untersucht. Die Verteilung des Zink über den Träger wurde mittels Schnittbildern im Elektronenmikroskop sowie in der Mikrosonde kontrolliert.

Ausgewählten Proben wurden außerdem noch mittels IR, $^{29}$Si-CP-MAS-NMR und EXAFS untersucht.

Die erfindungsgemäß erhältlichen Enolether der Formel I und die Dialkoxyverbindungen der Formel II sind wertvolle Zwischenprodukte zur Herstellung von Wirkstoffen und Riechstoffen. Insbesondere die Enolether sind begehrte Ausgangsstoffe z.B. zur Herstellung von γ, δ-ungesättigten Ketonen als Vorprodukte für die Herstellung von Isophytol.

Will man vor allem die Enolether gewinnen, kann man in an sich bekannter Weise die Verbindungen der Formel II durch Abspaltung von einem Mol $R^1OH$ in die entsprechenden Enolether der Formel I überführen. Dafür existieren zahlreiche aus DE-A-35 35 128, DE-A-37 22 891, DE-A-38 04 162, Chemical Abstracts, Vol. 94 (19); 156241f und DE-A-19544450 bekannte Verfahren.

Beispiel 1 bis 3

Tränkung mit Zinknitrat (20 % ZnO, 80 % $SiO_2$)

Eine Tränklösung, bestehend aus 365,52 g $Zn(NO_3)_2 \cdot 6\ H_2O$ (Merck) gelöst in 480 g destillierten Wasser wurde zwei Teile a 340 ml geteilt und 400 g $SiO_2$-Träger (Siligel, Fa. Solvay) bei Raumtemperatur mit dem ersten Anteil getränkt, der Präkatalysator anschließend für 16 h bei 120°C getrocknet und mit dem zweiten Anteil bei Raumtemperatur getränkt. Der noch feuchte Präkatalysator wurde dann in drei Portionen geteilt, die unter verschiedenen Bedingungen kalziniert worden sind (siehe Tabelle 1). Das molare $NO_3$/Zn-Verhältnis im Präkatalysator sind ebenfalls aus Tabelle 1 zu entnehmen.

In der oben beschriebenen Apparatur wurden ca. 90 ml des jeweiligen Präkatalysators eingefüllt. Propin/Allen-Gemisch (ca. 52 Vol.-%ig, 2,9 mmol/min) und Methanol (2,1 mmol/min; Gesamtlaufzeit mit Inerten: 7,8 mmol/min; Verhältnis MeOH/(Propin+Allen) = 0,71) wurden dann mittels HPLC-Pumpen zudosiert. Die Reaktionstemperatur betrug 170°C und der Druck 1,2 bar (abs, Partialdruck der Edukte: 0,77 bar). Nach der vollständigen Bildung des Katalysators (ca. 14 h) wurden die in Tabelle 1 angegebenen Selektivitäten beobachtet (Abkürzungen: 2 MP: 2-Methoxypropen; 2 DMP: 2,2-Dimethoxypropan; 1 MP: cis und trans 1-Methoxypropen; 1DMP: 1,1-Dimethoxypropan).

Tabelle 1

| # | Kalzinierung | | Präkatalysator $NO_3$/Zn | Selektivitäten | | | | | Umsatz Propin/ Allen |
|---|---|---|---|---|---|---|---|---|---|
| | T/°C | Zeit/h | | 2MP | 2DMP | Aceton | 1MP | 1DMP | |
| 1 | 120 | 16 | 0,33 | 81% | 10% | 2% | 5% | 1% | 37% |
| 2 | 250 | 4 | 0,61 | 83% | 9% | 1% | 5% | 1% | 39% |
| 3 | 320 | 2 | 0,17 | 83% | 8% | 1% | 6% | 1% | 27% |

Für den fertig gebildeten Katalysatoren wurden nach dem Ausbau die folgenden Werte ermittelt: BET-Oberfläche 180 - 210 $m^2$/g, Härte von 40 - 65 N/Formkörper. Das Verhältnis $NO_3$/Zn war bei den ausgebauten Katalysatoren immer <0,05.

Beispiel 4

Tränkung mit Zinkperchlorat (20 % ZnO, 80 % $SiO_2$)

Der Zn/$SiO_2$-Trägerkatalysator wurde durch Imprägnierung von röntgenamorphen $SiO_2$-Formkörpern (Kugeln mit ∅ 3-6 mm) mit einer BET-Oberfläche von 358 $m^2$/g, Wasseraufnahmekapazität von 0,9 ml/g, Härte von 43 N/Formkörper mit Zinkperchloratlösung, hergestellt.

100 g $SiO_2$-Träger (Siligel, Fa. Solvay) wurde mit 114,4 g $Zn(ClO_4)_2 . 6\ H_2O$ (Aldrich) gelöst in 30 g Wasser bei Raumtemperatur getränkt, der Präkatalysator anschließend 16 h bei 120°C getrocknet und dann bei 250°C 4h unter Luft kalziniert.

In die oben beschriebene Apparatur wurden ca. 90 ml des Präkatalysators eingefüllt. Propin/Allen-Gemisch (ca. 52 Vol.-%ig, 2,9 mmol/min) und Methanol (2,1 mmol/min; Gesamtzulauf mit Inerten: 7,8 mmol/min; Verhältnis MeOH/(Propin+Allen) = 0,71) wurden dann mittels HPLC-Pumpen zudosiert. Die Reaktionstemperatur betrug 170°C und der Druck 1,2 bar (abs), der Partialdruck der Edukte 0,77 bar. Nach der vollständigen Bildung des Katalysators (ca. 14 h) betrug der Propin/Allen-Umsatz 23 %. Als Hauptprodukte bildeten sich (Selektivitäten bzgl. Propin+Allen in Klammern) 2-Methoxypropen (79 %), 2,2-Dimethoxypropan (10 %), 1-Methoxypropen (6 %) und Aceton (3 %).

**Patentansprüche**

1. Verfahren zur Herstellung von Verbindungen der Formeln I bzw. II

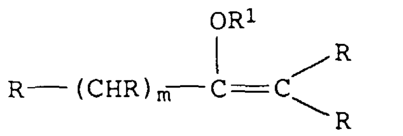 I 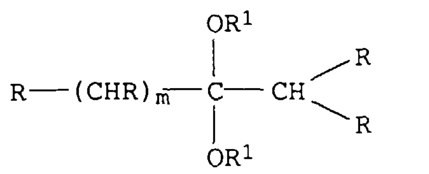 II

in denen $R^1$ Wasserstoff oder einen aliphatischen, cycloaliphatischen, araliphatischen, aromatischen oder heterocyclischen Rest oder einen Acylrest bedeutet, wobei diese Reste weitere Substituenten, die nicht mit Acetylenen oder Allenen reagieren, tragen können und die Reste R unabhängig voneinander für Wasserstoff, oder aliphatische, cycloaliphatische, araliphatische, aromatische oder heterocyclische Reste stehen, die unter Bildung eines Ringes miteinander verbunden sein können und m für 0 oder 1 steht, durch Addition von Verbindungen der Formel III

$$R^1OH \qquad \text{III}$$

an Acetylene oder Allene der Formeln IV bzw. V

$$R—C\equiv C—R \qquad \text{IV}$$

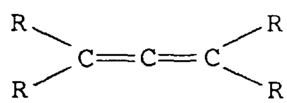 V

wobei $R^1$ und R die oben angegebenen Bedeutungen haben, in der Gasphase bei erhöhter Temperatur in Gegenwart eines heterogenen, durch Imprägnieren von Kieselsäure mit einem anorganischen Salz eines Elements der Gruppe 12 des Periodensystems der Elemente erhaltenen Katalysators, dadurch gekennzeichnet, daß man die Umsetzung bei einer Temperatur unterhalb von 200°C durchführt und einen Katalysator verwendet, der als aktiven Bestandteil ein röntgenamorphes Zinksilikat oder Cadmiumsilikat mit einem Gehalt an Zink bzw. Cadmium, berechnet als Oxid, von 1 bis 40 Gew.-% enthält, erhältlich durch Aufbringen eines Salzes von Zink oder Cadmium und einer anorganischen Oxosäure, das bei Temperaturen unterhalb von 400°C zersetzbar ist, auf amorphe Kieselsäure und Formierung des Katalysators vor der Umsetzung bei Temperaturen von 50 bis 500°C oder während der Umsetzung in situ bei Temperaturen von 50 bis 200°C, in Gegenwart einer Hydroxylgruppen enthaltenden Verbindung, ausgewählt aus der Gruppe bestehend aus Wasser, Alkanolen mit 1 bis 6 C-Atomen, Diolen oder Polyolen mit 2 bis 6 C-Atomen und 2 bis 3 OH-Gruppen oder Carbonsäuren mit 1 bis 6 C-Atomen.

**2.** Verfahren gemäß Anspruch 1, dadurch gekennzeichnet, daß man einen Katalysator verwendet, dessen katalytisch wirksame Komponente mit bis zu 80 Molprozent, bezogen auf Zink oder Cadmium, noch mit weiteren Metallen dotiert ist ausgewählt aus der Gruppe (A) bestehend aus Natrium, Kalium, Lithium, Rubidium, Cäsium, Beryllium, Magnesium, Calcium, Strontium, Barium, Mangan, Eisen, Kobalt, Nickel und Kupfer, und der Gruppe (B), bestehend aus Titan, Zirkon, Hafnium, Germanium, Zinn und Blei.

**3.** Verfahren gemäß Anspruch 1, dadurch gekennzeichnet, daß man einen Katalysator verwendet, der in Gegenwart des gleichen Alkanols formiert wurde, das mit dem Acetylen oder Allen umgesetzt werden soll.

**4.** Verfahren gemäß Anspruch 1, dadurch gekennzeichnet, daß man einen Katalysator verwendet, der durch Tränken von amorpher Kieselsäure mit Zinknitrat in wäßriger Lösung und anschließender Formierung erhältlich ist.

**5.** Verfahren gemäß Anspruch 1, dadurch gekennzeichnet, daß man die Addition der Hydroxylgruppen enthaltenden Verbindung $R^1OH$ an die Verbindungen der Formeln IV und V bei Temperaturen von 100 bis 200°C durchführt.

**6.** Verfahren gemäß Anspruch 1, dadurch gekennzeichnet, daß man 2-Methoxypropen und/oder 2,2-Dimethoxypropan durch Addition von Methanol an Methylacetylen und/oder Allen bei Temperaturen von 100 bis 200°C herstellt.

7. Verfahren zur Herstellung eines für das Verfahren gemäß Anspruch 1 geeigneten röntgenamorphen Zink- oder Cadmiumsilikats, dadurch gekennzeichnet, daß man Salze von Zink oder Cadmium und einer anorganischen Oxosäure, die bei Temperaturen unterhalb von 400°C zersetzbar sind, auf amorphe Kieselsäure durch trockenes Vermischen oder Tränken mit einer Lösung der Salze aufbringt und den Katalysator in Gegenwart von Hydroxylgruppen enthaltenden Verbindungen in einer Gas/Festkörperreaktion bei Temperaturen von 50 bis 500°C formiert.

8. Verfahren gemäß Anspruch 7, dadurch gekennzeichnet, daß man vor der Formierung des Katalysators durch die Gas/Festkörperreaktion bei 50 bis 500°C den durch Aufbringen des Zink- oder Cadmiumsalzes auf die amorphe Kieselsäure erhaltenen Präkatalysator einer Calzinierung bei 100 bis 400°C unterwirft, wobei die Temperatur und Verweilzeit so gewählt werden, daß der Präkatalysator noch mindestens 10 Mol-% des ursprünglichen Anions der Salze enthält.

9. Verfahren gemäß Anspruch 7, dadurch gekennzeichnet, daß man ein amorphes Zinksilikat durch Tränken von amorpher Kieselsäure mit einer Zinknitratlösung und anschließende Formierung gewinnt.

10. Röntgenamorpher Zink- oder Cadmiumsilikat-Katalysator, wie er gemäß dem Verfahren des Anspruchs 7 erhalten wird, der berechnet als Oxid 1 bis 40 Gew.-% Zink bzw. Cadmium enthält.

## Claims

1. A process for the preparation of compounds of the formulae I and II

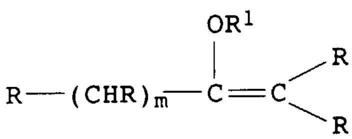 I 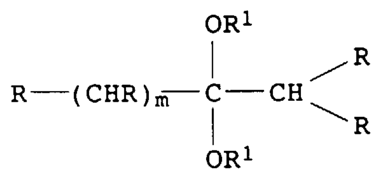 II

where $R^1$ is hydrogen or an aliphatic, cycloaliphatic, araliphatic, aromatic or heterocyclic radical or an acyl radical, it being possible for these radicals to carry further substituents which do not react with acetylenes or allenes, and the radicals R, independently of one another, are hydrogen or aliphatic, cycloaliphatic, araliphatic, aromatic or heterocyclic radicals which may be bonded to one another with the formation of a ring, and m is 0 or 1, by an addition reaction of a compound of the formula III

$$R^1OH \qquad III$$

with an acetylene or an allene of the formula IV or V

$$R—C\equiv C—R \qquad IV$$

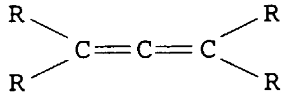 V

where $R^1$ and R have the abovementioned meanings, in the gas phase at elevated temperatures in the presence of a heterogeneous catalyst obtained by impregnating silica with an inorganic salt of an element of group 12 of the Periodic Table of Elements, wherein the reaction is carried out at below 200°C and the catalyst used is one which contains, as an active component, an X-ray amorphous zinc silicate or cadmium silicate containing from 1 to 40% by weight, calculated as oxide, of zinc or cadmium, obtainable by applying a salt of zinc or cadmium and an inorganic oxo acid, which salt is decomposable at below 400°C, to amorphous silica and forming the catalyst

before the reaction at from 50 to 500°C or during the reaction in situ at from 50 to 200°C in the presence of a hydroxyl-containing compound selected from the group consisting of water, alkanols of 1 to 6 carbon atoms, diols and polyols having 2 to 6 carbon atoms and 2 or 3 OH groups and carboxylic acids of 1 to 6 carbon atoms.

**2.** A process as claimed in claim 1, wherein the catalyst used is one whose catalytically active component is furthermore doped with up to 80 mol percent, based on zinc or cadmium, of further metals selected from the group (A) consisting of sodium, potassium, lithium, rubidium, cesium, beryllium, magnesium, calcium, strontium, barium, manganese, iron, cobalt, nickel and copper, and from the group (B) consisting of titanium, zirconium, hafnium, germanium, tin and lead.

**3.** A process as claimed in claim 1, wherein the catalyst used is one which was formed in the presence of the same alkanol which is to be reacted with the acetylene or allene.

**4.** A process as claimed in claim 1, wherein the catalyst used is one which is obtainable by impregnation of amorphous silica with zinc nitrate in aqueous solution and subsequent formation.

**5.** A process as claimed in claim 1, wherein the addition reaction of the hydroxyl-containing compound $R^1OH$ with the compounds of the formulae IV and V is carried out at from 100 to 200°C.

**6.** A process as claimed in claim 1, wherein 2-methoxypropene or 2,2-dimethoxypropane is prepared by an addition reaction of methanol with methylacetylene or allene at from 100 to 200°C.

**7.** A process for the preparation of an X-ray amorphous zinc silicate or cadmium silicate suitable for the process as claimed in claim 1, wherein salts of zinc or cadmium and an inorganic oxo acid which are decomposable at below 400°C are applied to amorphous silica by dry blending or impregnation with a solution of the salts, and the catalyst is formed in the presence of hydroxyl-containing compounds in a gas/solid reaction at from 50 to 500°C.

**8.** A process as claimed in claim 7, wherein, before the formation of the catalyst by the gas/solid reaction at from 50 to 500°C, the precatalyst obtained by applying the zinc or cadmium salt to the amorphous silica is subjected to a calcination at from 100 to 400°C, the temperature and the residence time being chosen so that the precatalyst still contains at least 10 mol% of the original anion of the salts.

**9.** A process as claimed in claim 7, wherein an amorphous zinc silicate is obtained by impregnation of amorphous silica with a zinc-nitrate solution and subsequent formation.

**10.** An X-ray amorphous zinc silicate or cadmium silicate catalyst as obtained by the process of claim 7, which contains from 1 to 40% by weight, calculated as oxide, of zinc or cadmium.

## Revendications

**1.** Procédé de fabrication de composés de formules I ou II

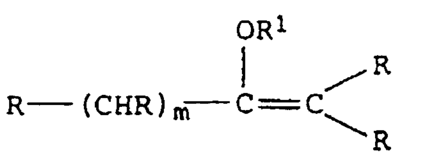 I

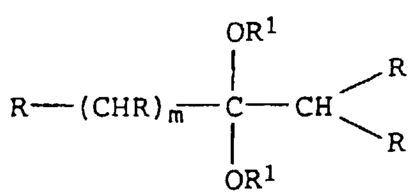 II

dans lesquelles $R^1$ représente un atome d'hydrogène, un groupe aliphatique, cycloaliphatique, araliphatique, aromatique ou hétérocyclique ou un groupe acyle, ces groupes pouvant contenir d'autres substituants ne réagissant pas envers les acétylènes ou allènes, les groupes R représentant indépendamment les uns des autres un atome d'hydrogène ou des groupes aliphatique, cycloaliphatique, araliphatique, aromatique ou hétérocyclique, qui peuvent être liés ensemble pour former un cycle, m vaut 0 ou 1,
par addition de composés de formule III

$$R^1OH \qquad III$$

à un acétylène ou un allène de formules IV ou V,

$$R—C{\equiv}C—R \qquad IV$$

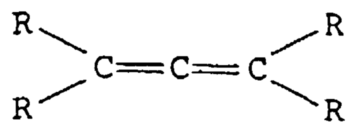 V

dans lesquelles R[1] et R ont les significations susmentionnées, en phase gazeuse, à température élevée et en présence d'un catalyseur hétérogène, obtenu par imprégnation de l'acide silicique avec un sel inorganique d'un élément du Groupe 12 du Tableau Périodique des Eléments, caractérisé en ce que l'on effectue la réaction à une température inférieure à 200°C et que l'on utilise un catalyseur contenant, en tant qu'élément actif, un silicate de zinc ou un silicate de cadmium amorphes aux rayons X, ayant une teneur en zinc ou cadmium de 1 à 40% en poids exprimé en oxyde, obtenus par application d'un sel de zinc ou de cadmium et d'un oxo-acide inorganique, qui se décompose à une température inférieure à 400°C, sur de l'acide silicique amorphe, puis la formation du catalyseur avant la réaction à des températures de 50°C à 500°C, ou pendant la réaction in situ à des températures allant de 50°C à 200°C, en présence d'un composé contenant des groupes hydroxyles, ce composé étant choisi dans un groupe constitué de l'eau, des alcanols de 1 à 6 atomes de carbone, des diols ou polyols de 2 à 6 atomes de carbone et de 2 à 3 groupes OH ou des acides carboxyliques de 1 à 6 atomes de carbone.

**2.** Procédé selon la revendication 1, caractérisé en ce qu'on utilise un catalyseur, dont les composants efficaces catalytiquement sont dopés par jusqu'à 80% en mole, par rapport au zinc et au cadmium, d'autres métaux choisis dans le groupe (A) composé du sodium, du potassium, du lithium, du rubidium, du césium, du béryllium, du magnésium, du calcium, du strontium, du baryum, du manganèse, du fer, du cobalt, du nickel et du cuivre et dans le groupe (B) composé du titane, du zirconium, de l'hafnium, du germanium, de l'étain et du plomb.

**3.** Procédé selon la revendication 1, caractérisé par l'utilisation d'un catalyseur, formé en présence du même alcanol qui doit réagir avec l'acétylène ou l'allène.

**4.** Procédé selon la revendication 1, caractérisé par l'utilisation d'un catalyseur obtenu par immersion d'un acide silicique amorphe dans du nitrate de zinc en solution aqueuse suivie de sa formation.

**5.** Procédé selon la revendication 1, caractérisé en ce que l'on met en oeuvre l'addition d'un composé $R^1OH$ contenant des groupes hydroxyles sur les composés de formules IV et V à des températures allant de 100°C à 200°C.

**6.** Procédé selon la revendication 1, caractérisé par la fabrication du 2-méthoxypropène et/ou du 2,2-diméthoxypropane par addition de méthanol sur du méthylacétylène et/ou de l'allène à des températures allant de 100°C à 200°C.

**7.** Procédé de fabrication d'un silicate de zinc ou de cadmium amorphe aux rayons X convenant pour le procédé selon la revendication 1, caractérisé en ce que l'on applique des sels de zinc ou de cadmium et d'un oxo-acide inorganique, décomposables à des températures inférieures à 400°C, sur de l'acide silicique amorphe par mélange à sec ou immersion avec une solution de ces sels, puis, on met en forme le catalyseur en présence des composés contenant des groupes hydroxyle, dans une réaction gaz/solide à des températures allant de 50°C à 500°C.

**8.** Procédé selon la revendication 7 caractérisé en ce qu'avant la formation du catalyseur par réaction gaz/solide à des températures comprises entre 50°C et 500°C, on soumet le précurseur de catalyseur, obtenu par application des sels de zinc ou de cadmium sur de l'acide silicique amorphe, à une calcination à des températures comprises entre 100°C et 400°C, la température et le temps de séjour étant choisis de façon que le précurseur de catalyseur contienne toujours au moins 10% en moles de l'anion initial des sels.

9. Procédé selon la revendication 7, caractérisé par l'obtention d'un silicate de zinc amorphe par immersion d'acide silicique amorphe dans une solution de nitrate de zinc, puis sa formation.

10. Catalyseur de silicate de zinc ou de cadmium amorphe aux rayons X, obtenu selon la revendication 7, contenant 1% à 40% en poids de zinc, respectivement de cadmium, exprimés en oxyde.

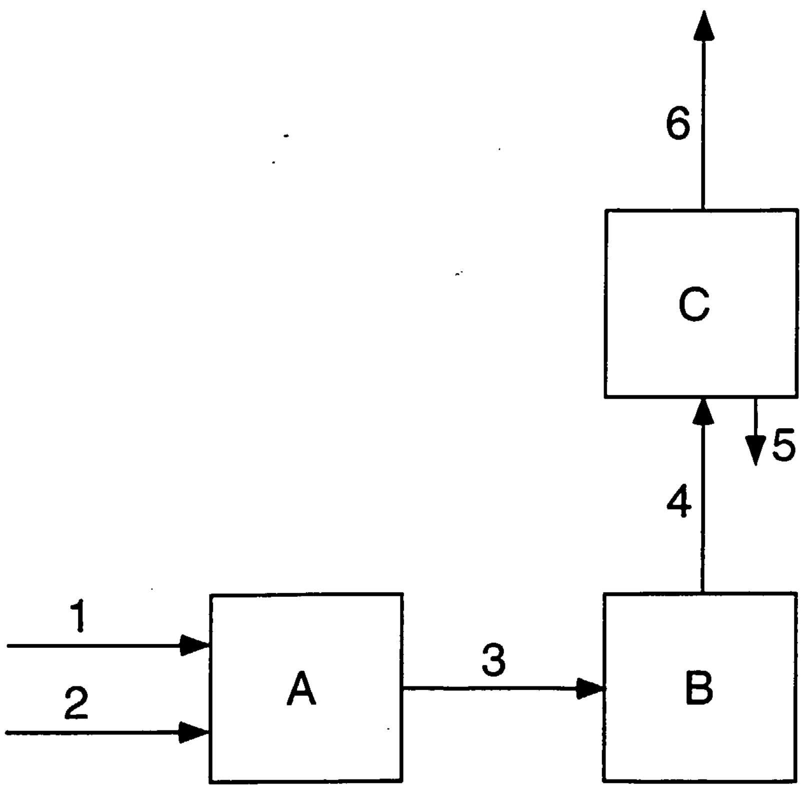
6
C
5
4
1
2
A
3
B